# EUROPÄISCHE PATENTANMELDUNG

(11) **EP 2 545 788 A1**
(43) Veröffentlichungstag der Anmeldung: **16.01.2013**
(21) Anmeldenummer: 11005726.2
(22) Anmeldetag: 13.07.2011
(51) Int. Cl.: A23L 1/29, A23L 1/30, A23L 1/302, A23L 1/303, A23L 1/304, A23L 1/305, A61K 33/06, A61K 33/10, A61K 33/14

(54) **Diätisches Mehrkomponentensystem**

(71) Anmelder: Hulliger, Martin, 8047 Zürich (CH)
(72) Erfinder: Hulliger, Martin, 8047 Zürich (CH)
(74) Vertreter: Grimm, Siegfried

(57) **Zusammenfassung**

Die Erfindung betrifft ein verbessertes Mehrkomponentensystem, enthaltend eine Grundkomponente und mindestens eine davon räumlich getrennte Zusatzkomponente wie in der Beschreibung definiert, sowie dessen Verwendung als Nahrungsergänzungsmittel und Pharmazeutikum.

## Beschreibung

Die Erfindung betrifft ein verbessertes Mehrkomponentensytem, enthaltend eine Grundkomponente und mindestens eine davon räumlich getrennte Zusatzkomponente, sowie dessen Verwendung als Nahrungsergänzungsmittel und Pharmazeutikum.

Es ist bekannt, dass der menschliche Organismus eine grosse Anzahl verschiedener Nährstoffe benötigt, darunter Minimum 60 Mineralien, 15 Vitamine, 12 essentielle Aminosäuren, 3 essentielle Fettsäuren.

Ferner ist bekannt, dass die Aufnahme von Mineralien pH - abhängig ist. Für einen geeigneten pH-Bereich lässt sich etwa folgende Reihenfolge angeben: Na, Mg > Ca, K > Mg, Fe > Zn, Cu > J; dies bedeutet, dass Natrium in einem sehr weiten pH-Bereich vom Organismus aufgenommen werden kann, wohingegen die Jodaufnahme nur in einem sehr engen pH-Bereich erfolgt.

Ferner ist bekannt, dass die Aufnahme anderer Nährstoffe, wie Vitamine, Aminosäuren, Fettsäuren, einer komplexen Wechselwirkung unterliegt und aufgrund biologischer Prozesse zu verschiedenen Tageszeiten unterschiedlich aktiv ist ("Biorhythmus").

An den menschlichen Bedarf angepasste Einkomponentensysteme und Mehrkomponentensysteme als Nahrungsergänzungsmittel oder Pharmazeutika sind bekannt und weit verbreitet.

So beschreibt Saltman et al (US5151274) eine Formulierung, auch in Form eines Mehrkomponentensystems, aus Kalzium und Spurenelementen zur Verbesserung des Knochenwachstums und Behandlung von altersbedingtem Knochenschwund.

Ferner beschreibt Collins (US2003/0018009) eine Formulierung mit erhöhtem Vitamin B12-Gehalt und deren Verwendung zur Behandlung von Autoimmun-Krankheiten.

Rüsing et al (WO2004/105516) beschreibt eine diätische Formulierung enthaltend mindestens eine n3 Fettsäure und mindestens einen Ballaststoff und deren Verwendung zur Gewichtskontrolle oder Gewichtsreduktion.

Die bekannten Formulierungen werden aus verschiedenen Gründen als ungenügend bzw. nachteilig angesehen. So sind diese oftmals auf sehr spezifische Wirkungen ausgerichtet und / oder in Ihrer Anwendung und / oder Handhabung eingeschränkt.

Es ist daher eine Aufgabe der vorliegenden Erfindung, die Nachteile der bekannten Formulierungen zu reduzieren. Insbesondere ist es eine Aufgabe der vorliegenden Erfindung, Formulierungen zur Verfügung zu stellen, die einen weiten Anwendungsbereich haben, leicht anzuwenden sind und / oder flexibel handhabbar sind.

Von besonderer Bedeutung ist, die Aufnahme wertvoller Nährstoffe zu verbessern um so einen positiven Einfluss auf den menschlichen Organismus zu nehmen. Vorteilhaft ist es ferner, ein flexibel handhabbares System bereitzustellen, um so speziellen Bedürfnissen gerecht zu werden. Eine weitere Aufgabe der vorliegenden Erfindung ist es, bestehende Therapiemöglichkeiten zu verbessern.

Die vorstehend umrissenen Aufgaben werden gemäss den unabhängigen Ansprüchen gelöst. Die abhängigen Ansprüche stellen vorteilhafte Ausführungsformen dar.

Sofern sich aus dem direkten Zusammenhang keine andere Bedeutung ergibt, haben die folgenden Begriffe die hier angegebene Bedeutung

Die im Zusammenhang mit der vorliegenden Erfindung gegebenen allgemeinen, bevorzugten und besonders bevorzugten Ausführungsformen, Bereiche usw. können beliebig miteinander kombiniert werden. Ebenso können einzelne Definitionen, Ausführungsformen usw. entfallen bzw. nicht relevant sein.

Der Begriff "umfassend" schliesst die Bedeutungen "enthaltend", "im wesentlichen enthaltend" und "bestehend aus" ein.

Der Begriff "kosmetische Behandlung", ist so zu verstehen, dass eine "therapeutische Behandlung" nicht mit umfasst ist. Entsprechend sind die Begriffe "kosmetische Formulierung" von einer "therapeutischen Formulierung" abzugrenzen.

Prozentangaben sind, soweit nicht anders vermerkt, als Massen-% angegeben.

Der Begriff "Mehrkomponentensystem" ist bekannt und umfasst Darreichungsformen, die zwei oder mehr individuelle (d.h. räumlich getrennte) Zusammensetzungen umfassen. Solche Mehrkomponentensysteme sind besonders geeignet, die einzelnen Komponenten unabhängig voneinander nach einem vorgegebenen Schema einzunehmen.

Nachfolgend wird die Erfindung, unter Bezugnahme auf die Figuren näher erläutert.

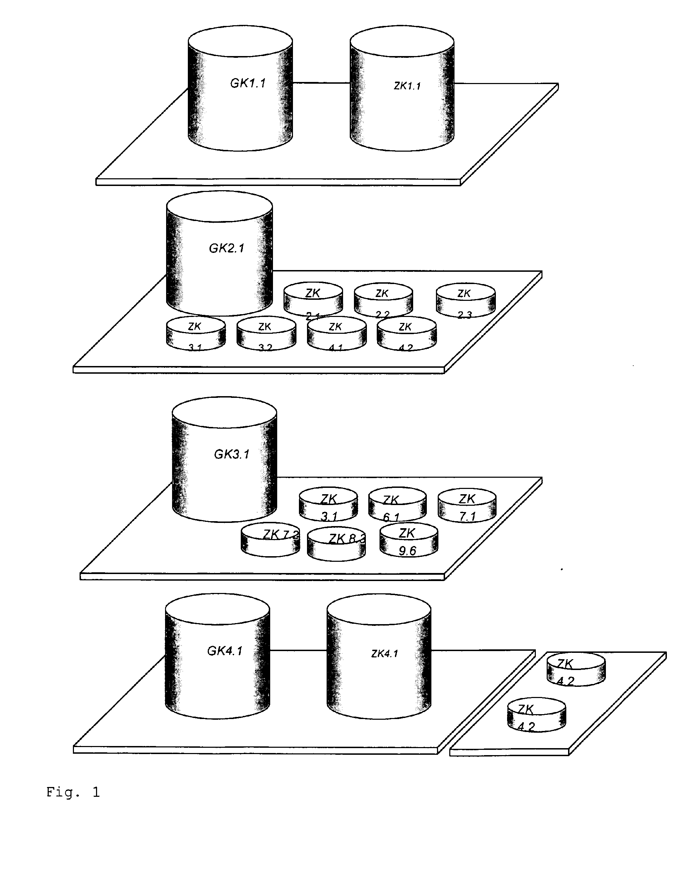

Fig. 1 zeigt in schematischer Weise verschiedene Ausführungsformen der erfindungsgemässen Mehrkomponentensystems; GK bezeichnet die Grundkomponente und ZK die Zusatzkomponente(n).

Fig. 1, oben, zeigt in einer einfachsten Ausführungsform des hier beschriebenen Mehrkomponentensystems eine Formulierung für Grund- und Zusatzkomponente in räumlich getrennten Formulierungen.

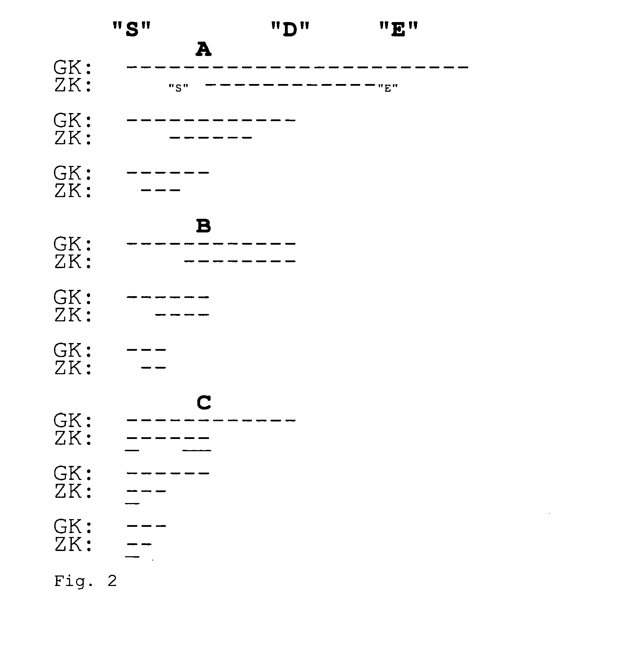

Fig. 1, 2. Reihe, zeigt in einer alternativen Ausführungsform des hier beschriebenen Mehrkomponentensystems eine Formulierung für Grund- und Zusatzkomponente in räumlich getrennten Formulierungen. In dieser Ausführungsform wird die GK individuell dosiert (bspw. als Pulver), die ZK ist bereits in gebrauchsfertigen vordosierten Formulierungen gegeben (bspw. als Brausetablette).

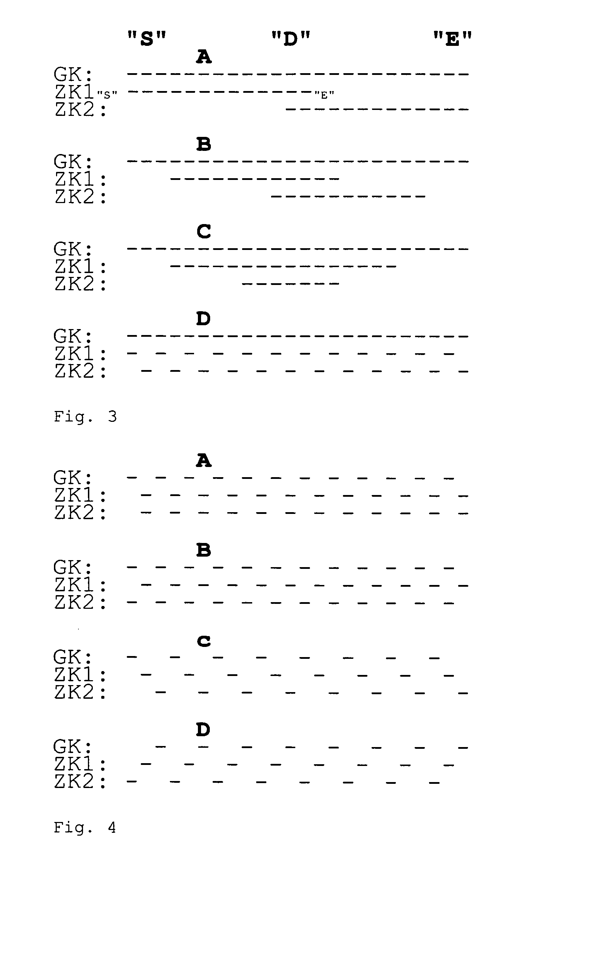

Fig. 1, 3. Reihe, zeigt in einer alternativen Ausführungsform des hier beschriebenen Mehrkomponentensystems eine Formulierung für eine Grundkomponente und zwei Zusatzkomponenten in räumlich getrennten Formulierungen.

In dieser Ausführungsform wird die GK individuell dosiert (bspw. als Pulver), die Zusatzkomponenten sind bereits in gebrauchsfertigen vordosierten Formulierungen gegeben (bspw. als Sirup).

Fig. 1, unten, zeigt in einer alternativen Ausführungsform des hier beschriebenen Mehrkomponentensystems eine Formulierung für eine Grundkomponente und eine Zusatzkomponenten in räumlich getrennten Formulierungen analog zur obersten Reihe. In dieser Ausführungsform wird zusätzlich eine weitere ZK individuell dosiert (bspw. als Pulver), diese Zusatzkomponente muss nicht zwingend den hier beschriebenen Zusatzkomponenten entsprechen, es kann sich dabei auch um ein Gas (bspw. Sauerstoff) oder ein pharmazeutisches Präparat handeln.

Fig. 2 zeigt in schematischer Weise verschiedene Einnahmepläne für eine Grundkomponente (GK) und eine oder mehrere Zusatzkomponente(n) (ZK). Ein Teilstrich symbolisert den Zeitraum vom ½ Monat. Innerhalb des Einnahmeinvervalls kann die Dosierung von GK und ZK konstant bleiben oder variieren. Angaben vorzugsweise von jedem genannten Punkt ("S" "D" "E") zu 30% ("S" "D" "E")+/-, 50% ("S" "D" "E") "+/-, 70%("S" "D" "E") +/- variabel, von "S"Start / "D"Dauer / "E"Ende der Gk+Zk(1).

In Fig. 2 A werden Einnahmepläne gezeigt, in denen das Grundintervall, also die Einnahmeperiode der GK, 12, bzw. 6 bzw. 3 Monate beträgt und die Einnahmeperiode der ZK 50 dieser Zeit beträgt.

In Fig. 2 B werden Einnahmepläne gezeigt, in denen das Grundintervall, 6 bzw. 3 bzw. 1.5 Monate beträgt und die Einnahmeperiode der ZK 2/3 dieser Zeit beträgt.

In Fig. 2 C werden Einnahmepläne gezeigt, in denen das Grundintervall, 6 bzw. 3 bzw. 1.5 Monate beträgt und die Einnahmeperiode der ZK 1/3 dieser Zeit beträgt.

Fig. 3 zeigt in schematischer Weise verschiedene Einnahmepläne für eine GK und mehrere ZK. Ein Teilstrich symbolisert den Zeitraum vom ½ Monat. Innerhalb des Einnahmeinvervalls kann die Dosierung von GK und ZK konstant bleiben oder variieren. Gemäss diesen Einnahmeplänen werden zwei ZK verabreicht, wobei diese ZK sequentiell verabreicht werden (3A), partiell überlappend verabreicht werden (3B), vollständig überlappend verabreicht werden (3C) oder alternierend verabreicht werden (3D). Dabei sind Angaben zu 5-95% von "S"+/-,"D"+/-,"E"+/-, der Gk/ZK1 variabel.

Fig. 4 zeigt in schematischer Weise verschiedene Einnahmepläne für eine GK und mehrere ZK. Ein Teilstrich symbolisert den Zeitraum vom ½ Monat. Innerhalb des Einnahmeinvervalls kann die Dosierung von GK und ZK konstant bleiben oder variieren. Gemäss diesen Einnahmeplänen werden zwei ZK verabreicht; dabei sind Angaben zu 5-95% von "S"+/-,"D"+/-,"E"+/-, der Gk/ZK1 variabel.

Die Erfindung betrifft in einem ersten Aspekt eine Formulierung / ein Produkt in Form eines Mehrkomponentensystems enthaltend eine Grundkomponente (GK) und davon räumlich getrennt eine oder mehrere Zusatzkomponenten (ZK), dadurch gekennzeichnet, dass die Grundkomponente basische Elektrolyte sowie ggf. Silikate enthält und die Zusatzkomponente Vitamine sowie ggf. Spurenelemente, ggf. Aminsäuren / Proteine, ggf. Pflanzenextrakte, ggf. weitere Inhaltsstoffe enthält.

Nachfolgend wird die erfindungsgemässe Formulierung, insbesondere dessen einzelne Komponenten, näher erläutert.

Es wurde überraschend gefunden, dass die separate Einnahme von Nährstoffen, ermöglicht durch die Auftrennung in Grundkomponente und Zusatzkomponente(n), zu einer Reihe von Vorteilen führt. So ist es bspw. möglich, mit der hier beschriebenen Formulierung / dem hier beschriebenen Produkt
■ eine weitaus höhere Nährstoffaufnahme zu erreichen, als dies mit bekannten Produkten möglich ist; und/oder
■ eine weitaus bessere Wirkung zu erzielen, als dies mit bekannten Produkten möglich ist; und/oder
■ eine speziell auf die Beseitigung eines unerwünschten Zustandes / die Therapie einer Krankheit adaptiertes Produkt zur Verfügung zu stellen; und/oder
■ positive Effekte zu erzielen, die nur durch die getrennte Einnahme von Nährstoffen ermöglicht wird; und/oder
■ die Wirkung von Medikamenten zu steigern und/oder
■ Medikamentenbedingte Schad/Abfallproduckte abzubauen/auszuscheiden/neutralisieren.

Ein wesentliches Element der vorliegenden Erfindung ist, dass die Formulierung in Form einer Grundkomponente und einer oder mehrerer Zusatzkomponenten vorliegt, wobei diese Komponenten voneinander getrennt sind. Ein derartiges System kann auch als "Kit of Parts" aus Grund- und Zusatzkomponente(n) beschrieben werden. Die beschriebene Aufteilung ermöglicht eine sequentielle und unabhängige Einnahme der Grund- und Zusatzkomponente(n). Diese wiederum ermöglicht eine Anpassung des Systems an den gewünschten Zweck (wie nachfolgend beschrieben) sowie die Verabreichung von an sich nicht kompatiblen Inhaltsstoffen. Die Begriffe Grund- und Zusatzkomponente sind nicht so zu verstehen, dass eine der beiden mengenmässig Grösser ist als die andere, noch das Handelsprodukte als "Grundkomponente" und "Zusatzkomponente" gekennzeichnet sind. Vielmehr sind diese Begriffe neutral als "eine erste Komponente" und "eine oder mehrere weitere Komponenten" zu verstehen.

Der Begriff "basischen Elektrolyte" ist bekannt und umfasst Alkalisalze und Erdalkalisalze, wie Carbonate, Hydrogencarbonate, Sulfate, Tartrate, Hydrogentartrate, sowohl wasserfrei als auch als Hydrate. Diese können fest und/oder flüssig vorliegen. Insbesondere sind Natriumcarbonat, Natriumhydrogencarbonat, Calciumcarbonat, Calciumhydrogencarbonat, Magnesiumcarbonat, Magnesiumhydrogencarbonat, Magnesiumsulfat, Natrium / Kaliumtartrat, Natriumtartrat, Kaliumtartrat umfasst. Besonders bevorzugt ist Natriumhydrogencarbonat. Die hier beschriebenen basischen Elektrolyte sind zu mehr als 50%, bevorzugt mehr als 66%, besonders bevorzugt mehr als 90% in der GK enthalten.

Der Begriff "Silikate" ist bekannt und umfasst synthetisch hergestellte Silikate als auch Silikate natürlichen Ursprungs, insbesondere aus der Gruppe der Diathomeerden.

Der Begriff "Vitamine" ist bekannt und umfasst insbesondere Vitamin A, Vitamine der B-Gruppe, Vitamin C, Vitamin D, Vitamin E, Vitamin K.

Die einzelnen Inhaltsstoffe können beliebig mit den Vitaminen kombiniert werden.

Der Begriff "Spurenelemente" ist bekannt und umfasst insbesondere Ca-, Cu-, Fe,- Mg-, Zn-, Mn-, Cr-, Mo-, Se-, Cl- und J- haltige Verbindungen.

Die Begriffe "Aminosäure" und "Protein" sind bekannt und umfassen die proteinogenen Aminosäuren, sowie andere für den menschlichen Verzehr bzw. pharmazeutische Anwendung geeignete Aminosäuren, Proteine und deren Metaboliten, insbesondere Carnitin, Kreatin, Taurin. Aminosäuren und Proteine können als isolierte Stoffe und / oder als Naturprodukte, reich an diesen Aminosäuren / Proteinen eingesetzt werden. Aminosäuren / Proteine können als einzelne Verbindung oder als Gemisch vorliegen.

Der Begriff "Pflanzenextrakte" ist bekannt und umfasst die für den menschlichen Verzehr bzw. pharmazeutische Anwendung geeignete Extrakte von Pflanzen aus Blättern, Blüten, Spross, Wurzel und / oder Frucht gewonnenen Auszüge, insbesondere wässrige oder alkoholische Auszüge. Bevorzugt sind Pflanzenextrakte ausgewählt aus der Gruppe umfassend Guarana, Coffein, Maca. Pflanzenextrakte können als isolierte Stoffe (bspw. Coffein) und / oder als Naturprodukte, reich an diesen Pflanzenextrakten (bspw. Grüntee-Extrakt) eingesetzt werden.

Der Begriff "weitere Inhaltsstoffe" umfasst solche Verbindungen die für den menschlichen Verzehr bzw. pharmazeutische Anwendung geeignet sind und sich nicht unter die vorstehend genannten Begriffe "Vitamine", "Spurenelemente", Aminsäuren / Proteine" oder "Pflanzenextrakte subsummieren lassen und einen positiven Effekt auf den menschlichen Körper bewirken. Der Begriff umfasst Aromastoffe, Duftstoffe, Hormone, hormonähnliche Verbindungen, insbesondere Hormone.

Die erfindungsgemässe Formulierung kann, muss aber nicht, in ihrer GK oder ZK weitere Lebensmittelzusatzstoffe enthalten. Der Begriff "Lebensmittelzusatzstoffe" sind aufgrund Ihrer "E-Nummer" allgemein bekannt und umfasst dabei insbesondere solche Stoffe, die die Haltbarkeit, Farbe, Geschmack, Verarbeitbarkeit positiv beeinflussen.

Wie bereits erwähnt, ist die erfindungsgemässe Formulierung als Nahrungsergänzungsmittel ("Supplement") und / oder als Pharmazeutikum geeignet. Es versteht sich, dass die Formulierung auf diese beabsichtigten Verwendungen entsprechend angepasst wird. Eine derartige Anpassung liegt im Rahmen des durchschnittlichen Wissens und Könnens einer Fachperson. Insbesondere können in der erfindungsgemässen Formulierung die Grundkomponente und die Zusatzkomponenten unabhängig voneinander als feste Darreichungsform und als flüssige Darreichungsform vorliegen. Ferner können die Darreichungsformen angepasst sein an orale oder transdermale Formulierungen.

Feste orale Darreichungsformen umfassen insbesondere Pulver, Tabletten, Kapseln.

Flüssige orale Darreichungsformen umfassen insbesondere Lösungen wie Getränke, Sirup, Suspensionen und Aerosole. Transdermale Darreichungsformen umfassen insbesondere Salben, Cremes, Öle.

Weiterhin können die erfindungsgemässen Formulierungen in magenresistenten Kapseln, in homöopatischen Formulierungen, Naturheilmitteln und / oder Notversorgungen aller Art enthalten sein.

Die Grund- und Zusatzkomponente der erfindungsgemässen Formulierung kann entweder direkt eingenommen werden, bspw. als Tablette oder in Form von Tropfen, oder sie kann in ein Endprodukt eingearbeitet werden, bspw. als Pulver oder Brausepulver in ein Getränk, als Snackprodukt oder in ein anderes Lebensmittel.

Die Gewichtsanteile der einzelnen Komponenten in GK und ZK können in weiten Bereichen variieren. In einer vorteilhaften Ausführungsform betrifft die Erfindung eine Formulierung wie hier beschrieben, dadurch gekennzeichnet, dass in der Grundkomponente 10-100 %, bevorzugt 50-99 % basischer Elektrolyt vorliegen und / oder dass in der Zusatzkomponente 0.01-500% der Tagesdosis vorliegen, die die WHO für dieses Vitamin empfiehlt; bevorzugt 30-300%, besonders bevorzugt 50-300%.

In einer bevorzugten Ausführung ist der Gehalt an Vitamin in der ZK 15 - 60% der von der WHO empfohlen Tagesdosis, sofern diese ZK als Brausetablette formuliert ist.

In einer weiteren bevorzugten Ausführung ist der Gehalt an Vitamin in der ZK 5 - 70 % der von der WHO empfohlenen Tagesdosis, sofern diese ZK nicht als Brausetablette formuliert ist.

In einer weiteren bevorzugten Ausführung ist eine Dosierung der Grundkomponente so vorgesehen, dass einer der basischen Elektrolyte, vorzugsweise Mengenelemente, über 300 mg pro Tag dosiert wird.

Die einzelnen Komponenten von GK und ZK sind bekannt und kommerziell erhältlich oder nach bekannten Methoden herstellbar.

Grundkomponente und Zusatzkomponente(n) können aus diesen einzelnen Komponenten nach bekannten Methoden der Lebensmitteltechnik und/oder der pharmazeutischen Technik hergestellt werden; bspw. durch mischen, dispergieren, lösen der einzelnen Komponenten. In einer weiteren vorteilhaften Ausführungsform betrifft die Erfindung eine Formulierung hergestellt nach den hier beschriebenen Verfahren.

Die Erfindung betrifft in einem zweiten Aspekt die Verwendung der erfindungsgemässen Formulierung als Nahrungsergänzungsmittel und / oder als Lebensmittel.

Die erfindungsgemässe Formulierung kann als Nahrungsergänzungsmittel verwendet werden, insbesondere um das Erreichen von Diätzielen zu unterstützen und / oder die Gesundheit des menschlichen Organismus in vielfältiger Weiser zu fördern. Nachfolgend wird dieser Aspekt der Erfindung näher erläutert.

Der Begriff "Nahrungsergänzungsmittel" ist bekannt und umfasst solche Zubereitungen, die der menschlichen Ernährung zugegeben werden können. Nahrungsergänzungsmittel sind geeignet zur erhöhten / verbesserten Versorgung des menschlichen Organismus mit den hier genannten Nährstoffen, und/oder zur unterstützenden Behandlung zur vorbeugung von negativen Zuständen.

Der Begriff "Lebensmittel" ist bekannt und umfasst solche Zubereitungen, die der menschlichen Ernährung dienen. Lebensmittel können in flüssiger Formulierung vorliegen, bspw. als alkoholfreies Getränk, als alkoholhaltiges Getränkt oder in fester Formulierung, bspw. als Snackprodukt oder Cerealien.

Die Erfindung betrifft ferner eine Formulierung wie hier beschrieben zur Erzielung eines kosmetischen (d.h. nicht-therapeutischen) Anti-Aging Effektes bzw. die Verwendung einer Formulierung wie hier beschrieben als Anti-Aging Mittel. Besonders vorteilhaft ist es für diese Anwendung, wenn die Zusatzkomponente Aminosäuren enthält, insbesondere ausgewählt aus der Gruppe umfasend Prolin (z.B. aus Gelatine, Muschelpulver, Molkeneiweiß, Käse, Weizenkeime, Orangensaft), Glycin (z.B. aus Gelatine, Muschelpulver, Molkeneiweiß, Hafer, Rindfleisch), Lysin (z.B. aus Gelatine, Weizenkeime, Amarant), Arginin (z.B. aus Gelatine, Weizenkeimen), Methionin (z.B. aus Molkeneiweiß, Hafer), Cystein (z.B. aus Hafer, Mais, Eiklar, Molkeneiweiß). In einer vorteilhaften Ausführung betrifft daher die vorliegende Erfindung ein Mehrkomponentensystem wie hier beschrieben in welchem die Zusatzkomponente Aminosäuren enthält, ausgewählt aus der Gruppe umfassend Prolin, Glycin, Lysin, Arginin, Methionin, Cystein.

Die Erfindung betrifft ferner eine Formulierung wie hier beschrieben zur kosmetischen (d.h. nicht-therapeutischen) Behandlung der Haare, einschliesslich Kräftigung der Haare, der Vermeidung von Haarausfall, dem verbesserten Wachstum der Haare bzw. die Verwendung einer Formulierung wie hier beschrieben als kosmetisches Haarbehandlungsmittel. Besonders vorteilhaft ist es für diese Anwendung, wenn die Zusatzkomponente Vitamine der B-Gruppe enthält, insbesondere ausgewählt aus der Gruppe umfassend Vitamin B2, B3, B5, B6, B7 und B12. In einer vorteilhaften Ausführung betrifft daher die vorliegende Erfindung ein Mehrkomponentensystem wie hier beschrieben in welchem die Zusatzkomponente Vitamine der B-Gruppe enthält, ausgewählt aus der Gruppe umfassend Vitamin B2, B3, B5, B6, B7 und B12, insbesondere B7. Besonders vorteilhaft ist es für diese Anwendung ferner, wenn die Zusatzkomponente Mineralien enthält, insbesondere ausgewählt aus der Gruppe umfassend Mg, Zn, Cu, Fe, I. In einer vorteilhaften Ausführung betrifft daher die vorliegende Erfindung ein Mehrkomponentensystem wie hier beschrieben in welchem die Zusatzkomponente Mineralien enthält, ausgewählt aus der Gruppe umfassend Mg, Zn, Cu, Fe und I.

Die Erfindung betrifft ferner eine Formulierung wie hier .beschrieben zur kosmetischen (d.h. nicht-therapeutischen) Behandlung der Haut, einschliesslich der Kopfhaut, bzw. die Verwendung einer Formulierung wie hier beschrieben als kosmetisches Hautbehandlungsmittel. Besonders vorteilhaft ist es für diese Anwendung, wenn die Zusatzkomponente Vitamine der E - Gruppe, insbesondere Tocopherol, enthält. In einer vorteilhaften Ausführung betrifft daher die vorliegende Erfindung ein Mehrkomponentensystem wie hier beschrieben in welchem die Zusatzkomponente Vitamine der E-Gruppe enthält, bevorzugt ausgewählt aus der Gruppe umfassend Tocopherol.

Die Erfindung betrifft ferner eine Formulierung wie hier beschrieben zur Unterdrückung der erneuten Gewichtszunahme nach erfolgreicher Diät (Jo-Jo Effekt) und / oder zur Verhinderung des Wiederaufbaus von Fettdepots bzw. die Verwendung einer Formulierung wie hier beschrieben zur Unterdrückung des Jo-Jo Effektes und / oder zur Verhinderung des Wiederaufbaus von Fettdepots. Besonders vorteilhaft ist es für diese Anwendung, wenn die Zusatzkomponente Aminosäuren enthält, insbesondere ausgewählt aus der Gruppe Arginin, Lysin, Ornithin. In einer vorteilhaften Ausführung betrifft daher die vorliegende Erfindung ein Mehrkomponentensystem wie hier beschrieben in welchem die Zusatzkomponente Aminosäuren enthält, ausgewählt aus der Gruppe umfassend Arginin, Lysin und Ornithin.

Die Erfindung betrifft ferner eine Formulierung wie hier beschrieben zur kosmetischen (d.h. nicht-therapeutischen) Stärkung der Muskulatur. Somit ist die hier beschriebene Formulierung als Ergänzung des allgemeinen Trainings (Sportlernahrung), als Ergänzung im Muskelaufbautraining (Bodybuilding) und / oder ergänzende Nahrung in Extremsituationen (Bergsteigen, Raumfahrt) geeignet. Besonders vorteilhaft ist es für diese Anwendung, wenn die Zusatzkomponente alle für den menschlichen Organismus wesentlichen Nährstoffanteile im richtigen Verhältnis, insbesondere wie hier beschrieben, enthält. In einer vorteilhaften Ausführung betrifft daher die vorliegende Erfindung ein Mehrkomponentensystem wie hier beschrieben in welchem die Zusatzkomponente Nährstoffe enthält, bevorzugt ausgewählt aus der Gruppe umfassend z.B. Basen, Proteine, Vitamine, Kohlenhydrate. Vorteilhaft sind diese Nahrstoffe auf mehrere ZK aufgeteilt.

Die Erfindung betrifft ferner eine Formulierung wie hier beschrieben zur Unterdrückung der erneuten Gewichtszunahme nach erfolgreicher Diät (Jo-Jo Effekt) und / oder zur Verhinderung des Wiederaufbaus von Fettdepots bzw. die Verwendung einer Formulierung wie hier beschrieben zur Unterdrückung des Jo-Jo Effektes und / oder zur Verhinderung des Wiederaufbaus von Fettdepots. Besonders vorteilhaft ist es für diese Anwendung, wenn die Zusatzkomponente Aminosäuren enthält, insbesondere ausgewählt aus der Gruppe Arginin, Lysin, Ornithin. In einer vorteilhaften Ausführung betrifft daher die vorliegende Erfindung ein Mehrkomponentensystem wie hier beschrieben in welchem die Zusatzkomponente Aminosäuren enthält, ausgewählt aus der Gruppe umfassend Arginin, Lysin und Ornithin.

Im Rahmen der vorliegenden Erfindung wird die erfindungsgemässe Formulierung / das erfindungsgemässe Nahrungsergänzungsmittel / das erfindungsgemässe Lebensmittel bevorzugt nach einem definierten Plan eingenommen; entsprechend kann die Formulierung / das Nahrungsergänzungsmittel / das Lebensmittel an diesen Plan angepasst werden. Ein solcher Einnahmeplan ist charakterisiert durch (i) die Dauer der Einnahme (z.B. 12 Monate), den Zeitpunkt der Einnahme (z.B. täglich) und die Dosierung (z.B. konstant oder ansteigend). Der Einnahmeplan wird auf das Behandlungsziel und die zu behandelnde Person abgestimmt. Der Einnahmeplan wird für die GK und jede ZK unabhängig voneinander in den hier beschriebenen Grenzen festgelegt. Falls mehr als eine ZK verabreicht werden, ist für die zweite und jede weitere ZK der Einnahmeplan völlig variabel und unabhängig vom Einnahmeplan für GK + ZK1.

Einnahmedauer: Gemäss Einnahmeplan kann die Einnahmedauer über einem breiten Bereich variieren, bevorzugt zwischen 1.5 und 12 Monate. Obwohl eine noch längere Behandlung möglich ist, hat es sich gezeigt, dass damit keine weitere Verbesserung erzielt wird. Ebenso führt eine kürzere Behandlung zu einer unvollständigen Wirkung. Vorteilhaft wird die Behandlung entweder (i) als Langzeitbehandlung ausgestaltet, typischerweise 9 - 15 Monate, oder (ii) als Intensiv-Behandlung, typischerweise 1.5 - 3 Monate, oder (iii) als Normal-Behandlung (welche besonders Vorteilhaft für Personen über 50 Jahre ist), typischerweise 4 - 8 Monate. Die Einnhamedauer kann auch kürzer sein als die o.g. 1.5 Monate; dies ist besonders dann vorteilhaft, wenn die erfindungsgemässen Formulierungen als Co-Therapie in Verbindung mit Medikamenten oder Naturheilmitteln verabreicht wird. Vorteilhaft wird die Behandlung dann (iv) als Co-Therapie zu Arzneimitteln / Naturheilmitteln ausgestaltet, typischerweise über einen Zeitraum von 1 Tag bis 2 Monate.

Die Einnahmedauer, wie hier beschrieben, bezieht sich auf die Grundkomponente ("Grundintervall"). Die Zusatzkomponente(n) werden ebenfalls im Zeitraum der Grundkomponente eingenommen, typischerweise aber nicht vor Beginn der Grundkomponente und nicht nach Beendigung der Grundkomponente. Werden mehrere Zusatzkomponenten eingesetzt, können deren Einnahmedauern unabhängig voneinander festgelegt werden, vgl Fig. 2.

Einnahmezeitpunkt: Gemäss Einnahmeplan kann der Einnahmezeitpunkt für GK und ZK über einen breiten Bereich variieren, wiederholt sich jedoch periodisch. So kann die Einnahme bspw. täglich, 2x täglich, 3x täglich, wöchentlich, monatlich erfolgen. Der Einnahmezeitpunkt von GK und ZK kann unabhängig voneinander festgelegt werden, bspw. GK 2xtäglich, ZK1 wöchentlich, ZK2 täglich.

Bei dem hier beschriebenen Protokoll ist es vorteilhaft, entweder (i) die GK 10 min - 24 Stunden, bevorzugt 30 min - 12 Stunden vor der ZK einzunehmen oder (ii) die GK 10 min - 24 Stunden, bevorzugt 30 min - 12 Stunden nach der ZK einzunehmen. Innerhalb des angewendeten Verwendungszwecks wird empfohlen, die Variante (i) oder (ii) beizubehalten.

Dosierung: Gemäss Einnahmeplan kann die Dosierung über einem breiten Bereich variieren. Grundsätzlich wird die Dosierung an das Behandlungsziel und die zu behandelnde Person angepasst. Die Dosierung für GK und ZK kann unabhängig voneinander entweder konstant oder variabel sein.

Bei dem hier beschriebenen Protokoll ist es vorteilhaft, die Dosierung der GK im Grundintervall konstant zu halten.

Bei dem hier beschriebenen Protokoll ist es vorteilhaft, die Dosierung der ZK im Grundintervall von einem Ausgangsniveau auf ein Maximalniveau zu erhöhen und dann wieder auf das Ausgangsniveau zu reduzieren. Das Maximalniveau kann dabei um den Faktor 2 - 5 über dem Ausgangsniveau liegen.

Bei dem hier beschriebenen Protokoll ist es möglich, eine oder mehrere (bspw. 2) ZK im Behandlungsintervall einzunehmen. Werden mehrere ZK eingenommen, können diese simultan, sequentiell oder alternierend eingenommen werden, vgl. Fig. 3.

Die Erfindung betrifft somit auch eine Formulierung / ein Nahrungsergänzungsmittel / ein Lebensmittel, angepasst an eine sequentielle Einnahme, wobei
■ die Grundkomponente täglich über einen Zeitraum von 12 Monaten und die Zusatzkomponente(n) täglich vom 2. - 11. Monat eingenommen wird oder
■ die Grundkomponente täglich über einen Zeitraum von 21 Wochen und die Zusatzkomponente(n) täglich von der 2. - 20. Woche eingenommen wird oder
■ die Grundkomponente täglich über einen Zeitraum von 4 Monaten und die Zusatzkomponente(n) wöchentlich über einen Zeitraum vom 4 Monaten oder
■ die Grundkomponente 2 x täglich über einen Zeitraum von 10 Wochen und die Zusatzkomponente(n) 1 x täglich von der 2. bis zur 9. Woche eingenommen wird.

Eine derartige sequentielle Einnahme verbessert die Wirksamkeit, insbesondere die hier beschriebenen kosmetischen Effekte, signifikant

Die Erfindung betrifft in einem dritten Aspekt die Verwendung der erfindungsgemässen Formulierung als Arzneimittel.

Es wird davon ausgegangen, dass ca. 10 Krankheitszustände auf den Mangel an je einer der eingangs erwähnten 90 Nährstoffe zurückzuführen sind. Dies bedeutet, dass ca. 900 Krankheiten durch eine geeignete Zufuhr dieser Nährstoffe, insbesondere durch die hier beschriebenen Mehrkomponentensysteme, therapiert werden können. Die Erfindung betrifft daher auch eine Formulierung wie hier beschrieben zur Verwendung als Pharmazeutikum. Nachfolgend wird dieser Aspekt der Erfindung näher erläutert.

Der Begriffe "Arzneimittel" bzw. "Pharmazeutikum" bzw. "pharmazeutische Zusammensetzung" sind bekannt und umfassen solche Zubereitungen, die dem Menschen verabreicht werden, um einen therapeutischen Zweck, insbesondere das Heilen, Vorbeugen, Verzögern und / oder Lindern eines pathologischen Zustandes (insbesondere einer Krankheit wie hier beschrieben), zu erzielen.

Die Erfindung betrifft ferner eine Formulierung wie hier beschrieben
■ zur Entschlackung (d. h. Ausscheidung von Giftstoffen und / oder schädlichen Stoffwechselprodukten aus dem menschlichen Organismus);
■ zur Behandlung von Azidose (Störung des Säure-Base Haushalts im menschlichen Organismus);
■ Zur Behandlung von Übergewicht;
■ zur Behandlung von Diabetes;
■ zur Behandlung von psychischen Erkrankungen (wie körperliche, emotionale, geistige Erschöpfung, Burnout);
■ zur Behandlung von Krebsleiden (d.h. Krankheiten, bei denen Körperzellen unkontrolliert wachsen, wie zur Behandlung von malignen Tumoren, zur Behandlung von bösartigen Hämoplastosen);
■ zur Behandlung von Herz-Kreislauf-Erkrankungen;
■ zur Behandlung von Hauterkrankungen (wie Psoriasis, Neurodermitis; Zellulitis, Cellulite, Akne);
■ zur Unterstützung der Wundheilung;
■ zur Behandlung von Gelenkserkrankungen (einschliesslich Rheumatische Beschwerden und Gicht);
■ zur Behandlung von Fehlfunktionen und Krankheiten der Leber;
■ zur Behandlung von Fehlfunktionen und Krankheiten der Nieren.
■ zur Behandlung Autoimunerkrankungen,
■ zur Behandlung von HIV
■ zur Behandlung von Allergien oder Intoleranzen (wie Lactose/Fruchtose Intoleranz).

Ohne sich an eine Theorie gebunden zu fühlen, wird davon ausgegangen, dass die o.g. Indikationen einen gemeinsames Merkmal aufweisen, da sie in durch Mängel an Nährstoffen (insbesondere Mineralien und Vitaminen) hervorgerufen oder verstärkt werden. Das erfindungsgemässe Arzneimittel kann auf spezifische Indikationen angepasst werden und ist somit in verschiedene Richtungen ausbaubar. Es zeigt sich in verschiedenen Tests, wie nachfolgend erläutert, dass das erfindungsgemässe Arzneimittel hoch wirksam ist, insbesondere wenn dass nachfolgend beschriebene Protokoll zur Einnahme angewandt wird.

Die Erfindung betrifft ferner die Verwendung einer Zusammensetzung wie hier beschrieben zur Herstellung eines Arzneimittels zur Behandlung einer Krankheit wie hier beschrieben.

Die Erfindung betrifft ferner eine pharmazeutische Zusammensetzung / Arzneimittel, umfassend eine Formulierung wie hier beschrieben.

Die Erfindung betrifft ferner eine Behandlungsmethode zur Behandlung des Menschen, umfassend den Schritt der Verabreichung einer therapeutisch wirksamen Menge eines Arzneimittels wie hier beschrieben.

Die erfindungsgemässe Formulierung ist ferner geeignet, bestehende Therapien für o.g. Erkrankungen zu ergänzen und zu verbessern; die Formulierungen sind somit geeignet als Co-Therapeutikum bzw. Co-Arzneimittel. Vielfach wird dabei ein synergistischer Effekt beobachtet, d.h. der Therapieerfolg steigt überproportional zu den entsprechenden Monotherapien (bestehend entweder nur durch Verabreichung des konventionellen Medikamentes oder nur durch Verabreichung der hier beschriebenen Formulierung). Somit betrifft die Erfindung ebenfalls eine Formulierung wie hier beschrieben als Co-Therapeutikum zur Behandlung der vorstehend genannten Krankheiten. Die Einnahme von bekanntem, konventionellen Arzneimittels und der hier beschriebenen Formulierung kann simultan oder sequentiell erfolgen.

Im Rahmen der vorliegenden Erfindung wird die erfindungsgemässe Formulierung / das erfindungsgemässe Arzneimittel vorteilhaft in einem definierten Einnahmeplan ("Protokoll") verabreicht; entsprechend kann, die Formulierung / das Arzneimittel an dieses Protokoll angepasst werden. Die Erfindung betrifft somit auch eine Formulierung / ein Arzneimittel, angepasst an eine sequentielle Einnahme, wobei
■ die Grundkomponente täglich über einen Zeitraum von 12 Monaten und die Zusatzkomponente(n) täglich vom 2. - 11. Monat verabreicht wird oder
■ die Grundkomponente täglich über einen Zeitraum von 21 Wochen und die Zusatzkomponente(n) täglich von der 2. - 20. Woche verabreicht wird oder
■ die Grundkomponente täglich über einen Zeitraum von 4 Monaten und die Zusatzkomponente(n) wöchentlich über einen Zeitraum vom 4 Monaten verabreicht wird oder
■ die Grundkomponente 2 x täglich über einen Zeitraum von 10 Wochen und die Zusatzkomponente(n) 1 x täglich von der 2. bis zur 9. Woche verabreicht wird.

Eine derartige sequentielle Verabreichung verbessert die Wirksamkeit, insbesondere die hier beschriebenen therapeutischen Effekte, signifikant.

Ein solches Protokoll ist charakterisiert durch (i) die Dauer der Einnahme (z.B. 12 Monate), den Zeitpunkt der Einnahme (z.B. täglich) und die Dosierung (z.B. konstant oder ansteigend). Das Protokoll wird auf das Behandlungsziel und die zu behandelnde Person abgestimmt. Ferner gelten die die im Zusammenhang mit dem zweiten Aspekt gemachten Ausführungen hier entsprechend.

So kann bei dem hier beschriebenen Protokoll die GK und jede ZK unabhängig voneinander in den hier beschriebenen Grenzen festgelegt werden.

Ferner ist es bei dem hier beschriebenen Protokoll vorteilhaft, entweder (i) die GK 10 min - 24 Stunden, bevorzugt 30 min - 12 Stunden vor der ZK einzunehmen oder (ii) die GK 10 min - 24 Stunden, bevorzugt 30 min - 12 Stunden nach der ZK einzunehmen. Innerhalb einer Therapie wird empfohlen, die Variante (i) oder (ii) beizubehalten.

Ferner ist es bei dem hier beschriebenen Protokoll vorteilhaft, die Dosierung der GK im Behandlungsintervall konstant zu halten.

Ferner ist es bei dem hier beschriebenen Protokoll vorteilhaft, die Dosierung der ZK im Behandlungsintervall von einem Ausgangsniveau auf ein Maximalniveau zu erhöhen und dann wieder auf das Ausgangsniveau zu reduzieren. Das Maximalniveau kann dabei um den Faktor 2 - 5 über dem Ausgangsniveau liegen.

Ferner ist es bei dem hier beschriebenen Protokoll möglich, eine oder mehrere (bspw. 2) ZK im Behandlungsintervall zu verabreichen. Werden mehrere ZK eingenommen, können diese simultan, sequentiell oder alternierend verabreicht werden.

Die nachstehend genannten Beispiele dienen der weiteren Erläuterung der Erfindung; sie sollen die Erfindung jedoch in keiner Weise limitieren.

### Beisp.: Diätisches Mehrphasen-Lebensmittel

Die Komponenten GK1, ZK1 und ZK2 werden wie nachfolgend beschrieben in Verbindung eingesetzt.

Die Komponenten GK1, ZK1 ZK2 können in beliebiger Formulierung verabreicht werden, bspw. als Pulver, Tablette, magenresistente Kabseln / Tabletten, in einen langsam auflösenden Trägerstoff beigemischt, ggf. Gelatine, Getränk, (z.B. Suspension / Sirup).

Bei den Mineralien wird der Gehalt auf das entsprechende Element bezogen; diese werden in Form handelsüblicher Salze eingesetzt und/oder können wie geschildert aufgearbeitet werden.

### GK1 pro 100g. Pulver

| | |
|---|---|
| Natron / Natiumbikarbonikum | 31 g. |
| Calcii Carbonas | 22,1 g. |
| Magnesii Carbonas Anhydricus | 17,6 g. |
| Terra Silicea Depurata | 10,4 g. |
| Magnesi Sulfas | 6,9 g. |
| Kali Nartrii Tartras | 4,7 g. |
| Natrii Sulfas Decahydricus | 1 g. |
| Mangani Carbonas Hydricus | 0,3 g. |
| L-Carnitin | 3 g. |
| Guarana- oder Grüner-Tee-Extrakt Pulver | 2 g. |

### ZK1 pro Tablette (Brausetabs):

| | | |
|---|---|---|
| Natriumhydrogencarbonat / bikarbonat | | 67 mg |
| Kohlenhydrate: ca. | | 641 mg |
| davon mehrwertige Alkohole | | 456 mg |
| Vitamine: | Vitamin A | 400 ug |
| | Vitamin D | 2,5 ug |
| | Vitamin E | 10 mg |
| | Vitamin C | 120 mg |
| | Thiamin | 1,4 mg |
| | Riboflavin | 1,6 mg |
| | B1 | 2 mg |
| | B2 | 2 mg |
| | B6 | 2 mg |
| | B12 | 1 ug |
| | Niacin | 18 mg |
| | Folsäure | 200 ug |
| | Biotine | 150 ug |
| | Pantothensäure | 6 mg |
| | Riboflavin | 0.6 mg |
| Mineralien: | Natrium | 115 mg |
| | Calcium | 140 mg |
| | Eisen | 4,2 mg |
| | Magnesium | 90 mg |
| | Zink | 4,5 mg |
| | Mangan | 1,5 mg |
| | Kupfer | 0,45 mg |
| | Chrom | 30 ug |
| | Molybdän | 30 ug |
| | Selen | 15 ug |
| | Chlor | 77 mg |
| | Jod | 60 ug. |

### ZK2 Zutaten pro 100 g.:

| | | |
|---|---|---|
| | Eiweiss | 83 g |
| | Kohlenhydrate | 2,4 g. |
| | Fett | 0,8 g. |
| | Lezithin | 5 g. |
| | Calciumortophosphat | 24 mg. |
| Vitamine: | Vitamin B1 | 2 mg. |
| | Vitamin B2 | 2 mg. |
| | Vitamin B6 | 1.9 mg. |
| | Vitamin B12 | 0.005 mg. |
| | Vitamin C | 75 mg. |
| | Vitamin E | 12 mg. |
| | Pantothenat | 8 mg. |
| | Folsäure | 0,45 mg. |
| | Niacin | 15 mg. |
| Mineralien: | Calcium | 1400 mg. |
| | E338 (Gehalt an P angegeben) | 700 mg. |
| | Zink | 4 mg. |

Proteine und/oder Aminosäuren: z.B. L-Glycin, L-Alanin, L-Valin, L-Leucin, L-Isoleucin, L-Prolin, L-Phenylalanin, L-Tyrosin, L-Triptophan, L-Serin, L-Threonin, L-Cystin, L-Methionin, L-Arginin, L-Histidin, L-Lysin, L-Asparaginsäure, L-Glutaminsäure, L- Carnitine, Kreatin, Kasein, Taurin.

Das diätische Mehrphasen-Lebensmittel wird zur Gewichtsreduktion verabreicht. In diesem Versuch wurden die Personen über den angegebenen Zeitraum und mit den angegebenen Komponenten, wie nachstehend beschrieben, behandelt.

| Komponente | 1. Monat | 2. Monat | 3. Monat | 4. Monat | 5. Monat |
|---|---|---|---|---|---|
| GK1.1 | 1ML | 2ML | 3ML | 4ML | 3ML |
| ZK1.1 | - | 2Tab | 3Tab | 2Tab | - |
| ZK2.1 | - | - | - | - | 2ML |
| | | | | | |

| Komponente | 1. Monat | 2. Monat | 3. Monat | 4. Monat | 5. Monat |
|---|---|---|---|---|---|
| GK1.1 | 1ML | 2ML | 1/2ML | 1/2ML | - |
| ZK1.1 | - | 2Tab | 3Tab | - | - |

| | | | | | |
|---|---|---|---|---|---|
| TL: 1 Messlöffel pro Tag, Tab: 1 Tablette pro Tag | | | | | |

Nach einer definierten Zeitspanne wurden die Versuchspersonen untersucht. Die Personen wurden beobachtet, bis Besserung eintrat. In bestimmten Zeitintervallen wurde die Vitalität der Personen überprüft.

In diesem Versuch wurden die nachstehenden Ergebnisse erzielt; ferner wurde in allen Fällen wurde ein Gesamtbefund von "gut" erkannt.

| Nr. / Geschlecht | Alter | Gewicht Beginn - Ende | Komponenten | Dauer |
|---|---|---|---|---|
| 1 / W | 35 | 75 - 57 | GK, ZK1 | 3 Mt. |
| 2 / W | 25 | 67 - 60 | GK, ZK1 | 3 Mt. |
| 3 / M | 25 | 90 - 79 | GK, ZK1 | 4 Mt. |
| 4 / W | 55 | 70 - 60 | GK, ZK1 | 4 Mt. |
| 5 / W | 35 | 70 - 58 | GK, ZK1C | 3 Mt. |
| 6 / W | 36 | 76 - 60 | GK, ZK1, ZK2 | 3 Mt. |
| 7 / W | 56 | 69 - 61 | GK, ZK1, ZK2 | 4 Mt. |
| 8 / M | 30 | 78 - 67 | GK, ZK1, ZK2 | 5 Mt. |
| 9 / W | 33 | 67 - 56 | GK, ZK1, ZK2 | 5 Mt. |
| 10 / M | 28 | 78 - 70 | GK, ZK1 | 4 Mt. |
| 11 / M | 36 | 81 - 72 | GK, ZK1 | 3 Mt. |
| 12 / W | 39 | 73 - 62 | GK, ZK1 | 3 Mt. |
| 12 / M | 45 | 85 - 73 | GK, ZK1 | 3 Mt. |

Die Ergebnisse zeigen, dass die erfindungsgemässe diätische Lebensmittel sowohl eine Gewichtsreduktion bewirkt als auch eine Verbesserung des allgemeinen Gesundheitszustandes / der Vitalität.

### Bsp. Behandlung Befindlichkeitsstörungen, Erkrankungen

Erfindgungsgemässe Mehrkomponentensysteme wurden ferner getestet zur Entschlackung und zur Behandlung von Cellulitis, Fettleibigkeit, Azidose , Rheuma/Gicht, Gelenk und Muskelschmerzen, Haarausfall, Herz / Kreislauf/ Haut Leiden, Krebs, Akne, Nierenleiden, Leberleiden, Entzündungen, Alkoholintoxikation ("Kater"), gegen infektionelle Erkrankungen, umweltbedingten Erkrankungen, altersbedingte Erkankungen, und zur Behandlung mentaler Störungen wie Stärkung der Konzentrationsfähigkeit, antidedressive Wirkung, Burnout.

In diesen Tests wurde das erfindungsgemässe Mehrkomponentensystem entweder alleine eingesetzt oder in Ergänzung zu Naturheilmitteln (wie Kräuterextrakten/ Omga-3 etc.) oder in Ergänzung zu Arzneimitteln (wie z.B. Antibiotika), um die Wirkungsweise zu überprüfen.

Es zeigte sich, dass die Naturheilmittel bzw. Arzneimittel eine verbesserte Wirkung zeigten (d.h. schneller, effizienter und/oder mit weniger Nebenwirkungen) und so eine verbesserte Therapie ermöglicht wurde.

Es zeigt sich ferner, dass es nach Testende, das Gewicht im Durchschnitt 2 Jahre gehalten wurde und der Gesundheitliche Zustand bei den Versuchspersonen als "Hervorragend" bewertet wird. Während dieser Zeit wurde keine zusätzliche körperliche Aktivität ausgeführt oder Einschränkungen bei der Nahrung gemacht. Somit wird ein Jo-Jo Effekt nicht beobachtet.

### Bsp.: Einnahmepläne

Bei dem Einnahmeplan sind Die Angaben von "Start"(S), "Dauer"(D) und "Ende"(E) des zu verabreichenden Intervall der "GK" und "ZK" in einem Bereich zwischen 5- 95% +/-, in Verbindung mit den aufgeführten Einnahmeprotokoll, bevorzugt, entweder 30% +/- und/oder 50% +/- und/oder 70% +/- variabel.

Die GK und ZK kann je nach Art der Verwendung wie vorstehend beschrieben angewendet werden.

Ab ZK2 ist diese nicht an die Vorgaben des Intervalls der GK / ZK1 gebunden und vom ersten Tag an einsetzbar.

Die ZK kann am gleichen Tag (wie hier beschrieben) oder auf einem darauf folgenden, Tag / Woche / Monat, beginnen, und ist nach einem oder mehreren Unterbrüchen wieder repetierbar / einsetzbar.

## Patentansprüche

1. Formulierung in Form eines Mehrkomponentensystems enthaltend eine Grundkomponente (GK) und davon räumlich getrennt eine oder mehrere Zusatzkomponenten (ZK), **dadurch gekennzeichnet dass**
■ die Grundkomponente basische Elektrolyte sowie ggf. Silikate enthält und
■ die Zusatzkomponente Vitamine sowie ggf. Spurenelemente, ggf. Aminsäuren / Proteine, ggf. Pflanzenextrakte enthält.

2. Formulierung nach Anspruch 1, in welcher
a. die basischen Elektrolyte ausgewählt sind aus der Gruppe umfassend Alkalisalze, der Erdalkalisalze und / oder
b. die Silikate ausgewählt sind aus der Gruppe umfassend der Diathomeerden und / oder
c. die Vitamine ausgewählt sind aus der Gruppe umfassend Vitamin A, B-Gruppe, C, D, E, K und/oder
d. die Spurenelemente ausgewählt sind aus der Gruppe umfassend Ca-, Fe,- Mg-, Zn-, Mn-, Cr-, Mo-, Se-, Cl- und J- haltige Verbindungen und/oder
e. die Aminosäuren / Proteine ausgewählt sind aus der Gruppe umfassend proteinogene Aminosäuren, Carnitin, Kreatin, Taurin und / oder
f. die Pflanzenextrakte ausgewählt sind aus der Gruppe umfassend Guarana, Coffein, Maca und Grüntee-Extrakt.

3. Formulierung nach Anspruch 1, **dadurch gekennzeichnet dass** die Grundkomponente und die Zusatzkomponente(n) unabhängig voneinander als feste Darreichungsform, insbesondere Pulver, Tablette, Kapsel und / oder als flüssige Darreichungsform, insbesondere Sirup, Suspension, Spray, vorliegen.

4. Formulierung nach Anspruch 1, **dadurch gekennzeichnet dass** in der Grundkomponente mehr als 30, bevorzugt mehr als 50 Massen-% basischer Elektrolyt vorliegen und / oder dass in der Zusatzkomponente 0.01 - 500 % Vitamine (bezogen auf die von der WHO empfohlene Tagesdosis) vorliegen.

5. Formulierung nach Anspruch 1 zur Verwendung als Nahrungsergänzungsmittel.

6. Formulierung nach Anspruch 5, angepasst an eine sequentielle Einnahme, wobei
■ die Grundkomponente täglich über einen Zeitraum von 12 Monaten und die Zusatzkomponente(n) täglich vom 2. - 11. Monat eingenommen wird oder
■ die Grundkomponente täglich über einen Zeitraum von 21 Wochen und die Zusatzkomponente(n) täglich von der 2. - 20. Woche eingenommen wird oder
■ die Grundkomponente täglich über einen Zeitraum von 4 Monaten und die Zusatzkomponente(n) wöchentlich über einen Zeitraum vom 4 Monaten oder
■ die Grundkomponente 2 x täglich über einen Zeitraum von 10 Wochen und die Zusatzkomponente(n) 1 x täglich von der 2. bis zur 9. Woche eingenommen wird.

7. Formulierung nach Anspruch 1 zur Verwendung als Pharmazeutikum.

8. Formulierung nach Anspruch 1 zur Behandlung von Übergewicht, Cellulitis und / oder Burn-out.

9. Formulierung nach Anspruch 7 oder 8, angepasst an eine sequentielle Einnahme, wobei
■ die Grundkomponente täglich über einen Zeitraum von 12 Monaten und die Zusatzkomponente(n) täglich vom 2. - 11. Monat eingenommen wird oder
■ die Grundkomponente täglich über einen Zeitraum von 21 Wochen und die Zusatzkomponente(n) täglich von der 2. - 20. Woche eingenommen wird oder
■ die Grundkomponente täglich über einen Zeitraum von 4 Monaten und die Zusatzkomponente(n) wöchentlich über einen Zeitraum vom 4 Monaten oder
■ die Grundkomponente 2 x täglich über einen Zeitraum von 10 Wochen und die Zusatzkomponente(n) 1 x täglich von der 2. - 9. Woche eingenommen wird.

10. Formulierung nach Anspruch 1, **dadurch gekennzeichnet dass** die Zusatzkomponente zusätzlich eine oder mehrere Arzneimittel enthält.

11. Nahrungsergänzungsmittel enthaltend eine Formulierung nach Anspruch 1.

12. Nahrungsergänzungsmittel nach Anspruch 11, wobei GK und ZK unabhängig voneinander in der Form von Pulver, Tablette, Sirup vorliegen.

13. Nahrungsergänzungsmittel nach Anspruch 11, angepasst an eine sequentielle Einnahme, wobei
■ die Grundkomponente täglich über einen Zeitraum von 12 Monaten und die Zusatzkomponente(n) täglich vom 2. - 11. Monat eingenommen wird oder
■ die Grundkomponente täglich über einen Zeitraum von 21 Wochen und die Zusatzkomponente(n) täglich von der 2. - 20. Woche eingenommen wird oder
■ die Grundkomponente täglich über einen Zeitraum von 4 Monaten und die Zusatzkomponente(n) wöchentlich über einen Zeitraum vom 4 Monaten oder
■ die Grundkomponente 2 x täglich über einen Zeitraum von 10 Wochen und die Zusatzkomponente(n) 1 x täglich von der 2. - 9. Woche eingenommen wird.

14. Lebensmittel, insbesondere in der Form eines Getränkes und / oder Snackproduktes, enthaltend eine Formulierung nach Anspruch 1.

15. Lebensmittel nach Anspruch 14, angepasst an eine sequentielle Einnahme, wobei
• die Grundkomponente täglich über einen Zeitraum von 12 Monaten und die Zusatzkomponente(n) täglich vom 2. - 11. Monat eingenommen wird oder
• die Grundkomponente täglich über einen Zeitraum von 21 Wochen und die Zusatzkomponente(n) täglich von der 2. - 20. Woche eingenommen wird oder
• die Grundkomponente täglich über einen Zeitraum von 4 Monaten und die Zusatzkomponente(n) wöchentlich über einen Zeitraum vom 4 Monaten oder
• die Grundkomponente 2 x täglich über einen Zeitraum von 10 Wochen und die Zusatzkomponente(n) 1 x täglich von der 2. - 9. Woche eingenommen wird.
